# EUROPEAN PATENT APPLICATION

(11) **EP 4 534 522 A1**
(43) Date of publication of application: **09.04.2025**
(21) Application number: 23815846.3
(22) Date of filing: 22.05.2023
(51) Int. Cl.: C07C 69/618, C07C 69/65, C09K 3/00

(54) **ADDITIVE**

(30) Priority: 31.05.2022 JP 2022088125
(71) Applicant: Miyoshi Oil & Fat Co., Ltd., Tokyo 124-8510 (JP)
(72) Inventor: NAKAMURA Daisuke, Tokyo 124-8510 (JP); KANEKO Kotaro, Tokyo 124-8510 (JP); KAWAI Koji, Tokyo 124-8510 (JP)
(74) Representative: Angerhausen, Christoph
(86) International application number: PCT/JP2023/018875
(87) International publication number: WO 2023/234087

(57) **Abstract**

Provided is an additive which exhibits a maximum absorption wavelength of 280-310 nm to efficiently absorb a UV-B region (280 to 320 nm), is suppressed in coloration during heating, has a high decomposition onset temperature during heating, and thus has excellent heat resistance. The additive according to the present invention is represented by the following general formula (1): 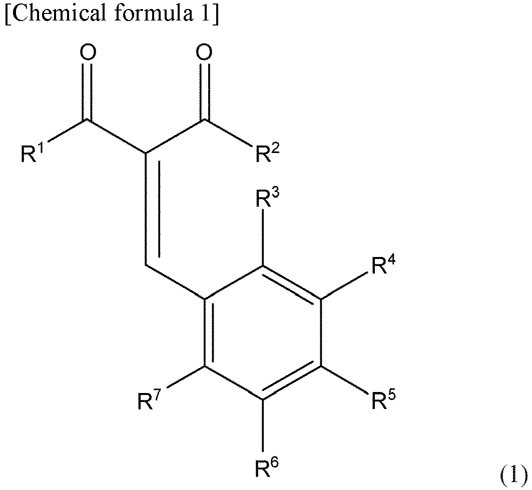 wherein, in the formula, R¹ and R² each independently represent an alkoxy group having 1 to 10 carbon atoms, or are bonded to a divalent group to form a ring, and R³ to R⁷ independently represent at least one selected from a hydrogen atom, a hydrocarbon group having 1 to 10 carbon atoms, a sulfur-containing group, a nitrogen-containing group, and a halogen, or any two among R³ to R⁷ are bonded to a divalent group to form a ring.

## Description

### Technical Field

The present invention relates to an additive.

### Background Art

A variety of additives have been used heretofore for imparting various functionalities to organic or inorganic materials. A known example of such includes an ultraviolet absorber for absorbing ultraviolet light to improve the light resistance of an organic material of, for example, resin, which is added to an organic or inorganic material to cut out a light having a specific range of wavelength. Examples of ultraviolet absorbers of organic compounds currently used include benzotriazole compounds, benzophenone compounds, triazine compounds, and malonic acid derivatives. Although the malonic acid derivative is known as an ultraviolet absorber for absorbing a short-wavelength UV-B region in ultraviolet light (see, for example, Patent Literatures 1 to 3), there is a demand for an ultraviolet absorber that more efficiently absorbs a UV-B region and, for example, when added to a resin, suppresses coloration when the resin is thermally processed and exhibits a high decomposition onset temperature when the resin is heated; that is, there is a demand of an ultraviolet absorber that is excellent in heat resistance, has a good appearance, and does not lose its ultraviolet absorbing capability.

### Citation List

### Patent Literature

PLT 1: JP-A-2022-052594
PLT 2: JP-A-2021-181528
PLT 3: JP-A-S50-008844

### Summary of Invention

### Technical Problem

The present invention has been made in view of the above circumstances, and it is an object of the present invention to provide an additive that exhibits a maximum absorption wavelength of 280 to 310 nm for efficiently absorbing a UV-B region (280-320nm), suppresses coloration during heating, has a high decomposition onset temperature during heating, and thus has excellent heat resistance.

### Solution to Problem

To solve the above-mentioned object, the additive of the present invention is represented by the following general formula (1):

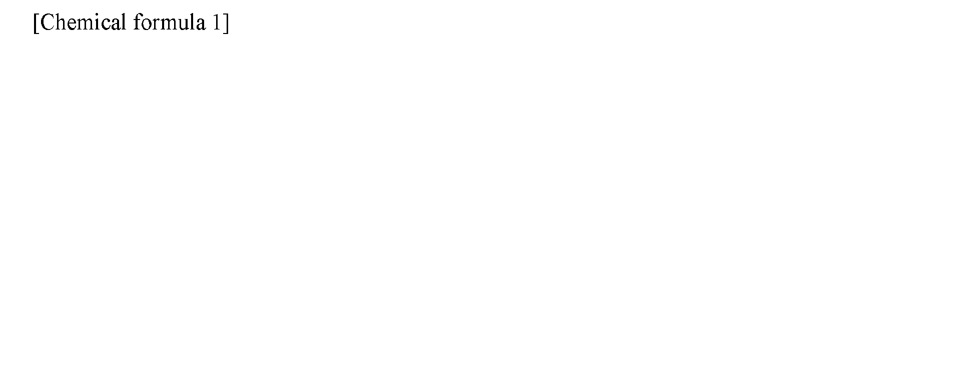

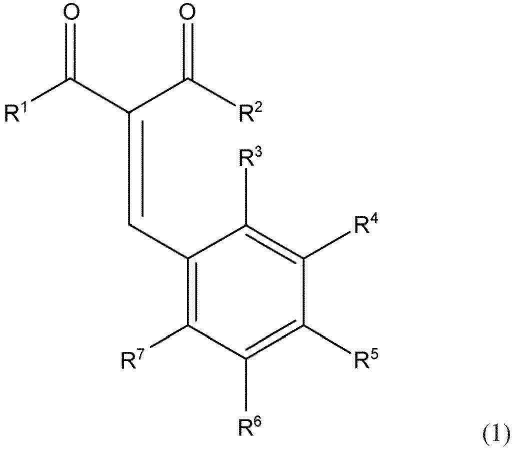

In the formula, R¹ and R² each independently represent an alkoxy group having 1 to 10 carbon atoms, or are bonded to a divalent group to form a ring. R³ to R⁷ independently represent at least one selected from a hydrogen atom, a hydrocarbon group having 1 to 10 carbon atoms, a sulfur-containing group, a nitrogen-containing group, and a halogen, or any two among R³ to R⁷ are bonded to a divalent group to form a ring.

The ultraviolet absorber of the present invention is characterized by an additive represented by the above-defined general formula (1).

### Advantageous Effects of Invention

The additive of the present invention exhibits a maximum absorption wavelength of 280 to 310 nm, which allows efficient absorption of a UV-B region, and the additive also suppresses coloration during heating, has a high decomposition onset temperature during heating, and thus has excellent heat resistance.

### Description of Embodiments

Specific embodiments of the present invention will be described in detail hereunder.

The number of carbon atoms as used herein is an integer.

### (Additive)

The additive according to the present invention is represented by the above-defined general formula (1).

In the formula (1), R¹ and R² each independently represent an alkoxy group having 1 to 10 carbon atoms, or are bonded to a divalent group to form a ring. It is preferred that each of R¹ and R² be an alkoxy group having 1 to 10 carbon atoms.

The hydrocarbon group forming the alkoxy group is preferably an aliphatic hydrocarbon group, more preferably a saturated aliphatic hydrocarbon group. The group may be linear or branched.

Examples of the alkoxy group include, but are not particularly limited to, a methoxy group, an ethoxy group, a propoxy group, a 1-methylethoxy group, a butoxy group, a 2-methyl-1-propoxy group, a 2-methyl-2-propoxy group, a pentyloxy group, a hexyloxy group, a heptyloxy group, an octyloxy group, a nonyloxy group, and a decanyloxy group. It is preferred that the alkoxy groups have 1 to 4 carbon atoms, among which a methoxy group, an ethoxy group, a propoxy group, a 1-methylethoxy group, a butoxy group, a 2-methyl-1-propoxy group, and a 2-methyl-2-propoxy group are more preferred, and a methoxy group, an ethoxy group, a propoxy group, and a 1-methylethoxy group having 1 to 3 carbon atoms are even more preferred, of which a methoxy group having one carbon atom is particularly preferred.

Examples of the divalent group forming a ring include, but are not particularly limited to, a hydrocarbon group, an oxygen-containing group, a nitrogen-containing group, and a sulfur-containing group.

Examples of the hydrocarbon group include groups of those listed as monovalent hydrocarbon groups of R³ to R⁷ in the following from which one hydrogen atom is removed. Of these, it is preferred that the hydrocarbon group be an aliphatic hydrocarbon group, more preferably a saturated aliphatic hydrocarbon group. The group may also be linear or branched.

The oxygen-containing group has, for example, 1 to 10 carbon atoms. Examples of the oxygen-containing group having 1 to 10 carbon atoms include, for example, an ether group-containing group, an ester group-containing group, a carbonyl group-containing group, and a carboxy group-containing group.

The nitrogen-containing group has, for example, 1 to 10 carbon atoms. Examples of the nitrogen-containing group having 1 to 10 carbon atoms include, for example, a primary, secondary or tertiary amino group (including the one that forms an amide group (-NHCO-), an imide group (-CONRCO-), or a urea group (-NHCONH-) with the above-mentioned bonding at a site containing an end of divalent group), an amide group-containing group, an imide group-containing group, a urea group-containing group, and a urethane group-containing group.

The sulfur-containing group has, for example, 1 to 10 carbon atoms. Examples of the sulfur-containing group having 1 to 10 carbon atoms include a thioether group-containing group, a sulfide group-containing group, a disulfide group-containing group, a thioester group-containing group, a thioamide group-containing group, a sulfonyl group-containing group, a thiocarbonyl group-containing group, a thiourea group-containing group, a thiocarbamate group-containing group, and a dithiocarbamate group-containing group.

Of these, it is preferred that the group be a group that forms an amide group, an imide group, or a urea group with the above-mentioned bonding at a site including an end of divalent group, and has 1 to 5 carbon atoms; and it is more preferred that the group be a group that forms a urea group with the above-mentioned bonding.

In the formula (1), R³ to R⁷ independently represents at least one selected from a hydrogen atom, a hydrocarbon group having 1 to 10 carbon atoms, a sulfur-containing group, a nitrogen-containing group, and a halogen, or any two among R³ to R⁷ are bonded to a divalent group to form a ring.

Examples of the hydrocarbon group include a saturated or unsaturated aliphatic hydrocarbon group, an alicyclic hydrocarbon group, and an aromatic hydrocarbon group.

Examples of the saturated or unsaturated aliphatic hydrocarbon group include an alkyl group, an alkenyl group, and an alkynyl group having 1 to 10 carbon atoms, and among these, a linear or branched alkyl group is preferred.

Examples of the linear or branched alkyl group include, but are not particularly limited to, a methyl group, an ethan-1-yl group, a propan-1-yl group, a 1-methylethan-1-yl group, a butan-1-yl group, a 1-methylpropan-1-yl group, a 2-methylpropan-1-yl group, a 2-methylpropan-2-yl group, a pentan-1-yl group, a pentan-2-yl group, a hexane-1-yl group, a heptan-1-yl group, an octan-1-yl group, a nonan-1-yl group, and a decan-1-yl group.

The alicyclic hydrocarbon group preferably has 3 to 10, more preferably 3 to 6 carbon atoms, and examples of the alicyclic hydrocarbon group include, but are not particularly limited to, a cyclopropyl group, a cyclobutyl group, a cyclopentyl group, a cyclohexyl group, a cycloheptyl group, a cyclooctyl group, and groups containing these groups as skeletons.

The aromatic hydrocarbon group includes an aromatic ring such as benzene ring, naphthalene ring, and anthracene ring, and has, preferably, 6 to 10 carbon atoms. Examples of the aromatic hydrocarbon group include, but are not particularly limited to, a phenyl group, a 2-methylphenyl group, a 3-methylphenyl group, a 4-methylphenyl group, a 2,4-dimethylphenyl group, a 2,5-dimethylphenyl group, a 3,4-dimethylphenyl group, a 3,5-dimethylphenyl group, a 2,4,5-trimethylphenyl group, a 2,4,6-trimethylphenyl group, a 1-naphthyl group, a 2-naphthyl group, and groups containing these groups as skeletons.

Examples of the sulfur-containing group include, but are not particularly limited to, a thiophene group-containing group, a thiazole group-containing group, a thiol group-containing group, a thioether group-containing group, a thioalkoxy group-containing group, a sulfo group-containing group, a sulfide group-containing group, a disulfide group-containing group, a thioester group-containing group, a thioamide group-containing group, a sulfonyl group-containing group, a thiocarbonyl group-containing group, a thiourea group-containing group, a thiocarbamate group-containing group, and a dithiocarbamate group-containing group, and the number of carbon atoms is preferably 0 to 10.

Examples of the nitrogen-containing group include, but are not particularly limited to, a cyano group-containing group, a cyanate group-containing group, an isocyanate group-containing group, a nitro group-containing group, a nitroalkyl group-containing group, an amide group-containing group, a urea group-containing group, a urethane group-containing group, an imide group-containing group, a carbodiimide group-containing group, an azo group-containing group, a pyridine group-containing group, an imidazole group-containing group, an amino group-containing group (a primary amino group-containing group, a secondary amino group-containing group, a tertiary amino group-containing group), and an aminoalkyl group-containing group, and the number of carbon atoms is preferably 0 to 10.

Examples of the halogen include, but are not particularly limited to, a fluorine atom, a chlorine atom, a bromine atom, and an iodine atom.

Of these, it is preferred that each of R³ to R⁷ be a hydrogen atom, a hydrocarbon group, or a halogen. A combination of hydrogen atom(s) and hydrocarbon group(s) or a combination of hydrogen atom(s) and halogen(s) is particularly preferred.

It is particularly preferred that the hydrocarbon group be an aliphatic hydrocarbon group, more preferably a saturated aliphatic hydrocarbon group. Such hydrocarbon group has 1 to 10 carbon atoms, preferably 1 to 8 carbon atoms, and more preferably 1 to 5 carbon atoms.

When any two of R³ to R⁷ are bonded to a divalent group to form a ring, examples of the divalent group forming a ring include, but are not particularly limited to, a hydrocarbon group, an oxygen-containing group, a nitrogen-containing group, and a sulfur-containing group. Specifically, reference is made to the above-mentioned list shown as divalent groups to which R¹ and R² are bonded to form a ring.

It is preferred in terms of efficiently absorbing a UV-B region that the additive of the present invention exhibits a maximum absorption wavelength of 280 to 310 nm, more preferably 280 to 300 nm, and even more preferably 280 to 290 nm in a 100 µM solution. The maximum absorption wavelength may be measured using, for example, chloroform or dimethylsulfoxide as a solvent.

A particularly preferable example of the additive of the present invention exhibits a maximum absorption wavelength of 300 nm or less in a 100 µM solution.

In this respect, each of R³ to R⁷ of the additive of the present invention is preferably a hydrogen atom, a hydrocarbon group, or a halogen. Of these hydrocarbon groups, it is preferred that such hydrocarbon group be an aliphatic hydrocarbon group, more preferably a saturated aliphatic hydrocarbon group. Such hydrocarbon group has 1 to 10 carbon atoms, preferably 1 to 8 carbon atoms, and more preferably 1 to 5 carbon atoms. The halogen is preferably a chlorine atom or a fluorine atom, and more preferably a chlorine atom. It is also preferred that at least R⁵ be a halogen.

In addition, it is preferred that R³ to R⁷ be composed of hydrogen atom(s) and hydrocarbon group(s) of which at least one of them is a hydrogen atom and at least another one of them is a hydrocarbon group, or that R³ to R⁷ be composed of hydrogen atom(s) and halogen(s) of which at least one of them is a hydrogen atom and at least another one of them is a halogen. It is more preferred that R³ to R⁷ be composed of hydrogen atom(s) and halogen(s) of which at least one of them is a hydrogen atom and at least another one of them is a halogen.

The additive of the present invention has excellent heat resistance from the two viewpoints of: coloration caused by oxidation and heat in an oxygen atmosphere during heating; and decomposition caused only by heat in a nitrogen atmosphere (decomposition onset temperature).

It is preferred from the viewpoint of suppressing coloration during heating (heat resistance) that each of R³ to R⁷ be a hydrogen atom, a hydrocarbon group, or a halogen, and more preferably a hydrogen atom or a halogen. The halogen is preferably a chlorine atom or a fluorine atom, and more preferably a chlorine atom. It is also preferred that at least R⁵ be a halogen.

Further, it is preferred that R³ to R⁷ be composed of hydrogen atom(s) and hydrocarbon group(s) of which at least one of them is a hydrogen atom and at least another one of them is a hydrocarbon group, or that R³ to R⁷ be composed of hydrogen atom(s) and halogen(s) of which at least one of them is a hydrogen atom and at least another one of them is a halogen. It is more preferred that R³ to R⁷ be composed of hydrogen atom(s) and halogen(s) of which at least one of them is a hydrogen atom and at least another one of them is a halogen.

It is preferred in terms of heat resistance that the additive of the present invention have a decomposition onset temperature of 190°C or higher, more preferably 200°C or higher, and even more preferably 210°C or higher during heating. In view of this respect, each of R³ to R⁷ is preferably a hydrogen atom, a hydrocarbon group, or a halogen. Particularly, it is preferred that R³ to R⁷ be composed of hydrogen atom(s) and hydrocarbon group(s) of which at least one of them is a hydrogen atom and at least another one of them is a hydrocarbon group, or that R³ to R⁷ be composed of hydrogen atom(s) and halogen(s) of which at least one of them is a hydrogen atom and at least another one of them is a halogen. Of these, it is preferred that such hydrocarbon group be an aliphatic hydrocarbon group, more preferably a saturated aliphatic hydrocarbon group. Such hydrocarbon group have 1 to 10 carbon atoms, preferably 1 to 8 carbon atoms, more preferably 1 to 5 carbon atoms, and even more preferably 2 to 5 carbon atoms.

The additive of the present invention may be of a solid or a liquid state at 25°C but the additive of solid state at 25°C exhibits favorable handleability when it is added or mixed into a resin, and therefore is preferred.

In view of this respect, each of R³ to R⁷ is preferably a hydrogen atom, a hydrocarbon group, or a halogen. Particularly, it is preferred that R³ to R⁷ be composed of hydrogen atom(s) and hydrocarbon group(s) of which at least one of them is a hydrogen atom and at least another one of them is a hydrocarbon group, or that R³ to R⁷ be composed of hydrogen atom(s) and halogen(s) of which at least one of them is a hydrogen atom and at least another one of them is a halogen. Of these, it is preferred that such hydrocarbon group be an aliphatic hydrocarbon group, more preferably a saturated aliphatic hydrocarbon group. It is preferred that the hydrocarbon groups of R³ to R⁷ have a quaternary carbon, or that three or more of R³ to R⁷ are hydrocarbon groups.

Examples of the additives of the present invention include, but are not particularly limited to, a benzylidene dialkyl malonate-based compound and a benzylidene barbituric acid-based compound. Examples of the benzylidene dialkyl malonate-based compound include, but are not particularly limited to, dimethyl 2-(4-chlorobenzylidene)malonate, dimethyl 2-(4-fluorobenzylidene)malonate, dimethyl 2-(4-ethylbenzylidene)malonate, dimethyl 2-(4-isopropylbenzylidene)malonate, dimethyl 2-(4-tert-butylbenzylidene)malonate, dimethyl 2-(4-isobutylbenzylidene)malonate, dimethyl 2-(2,4,6-trimethylbenzylidene)malonate, diisopropyl 2-(4-isopropylbenzylidene)malonate, and diisopropyl 2-(3,4-(methylenedioxy)benzylidene)malonate. Examples of the benzylidene barbituric acid-based compound include, but are not particularly limited to, 5-(4-chlorobenzylidene) barbituric acid. Of these, it is preferred in terms of efficient absorption of a UV-B region (280 to 320 nm) and heat resistance that the compound be a benzylidene dialkyl malonate, and it is more preferred that the compound be dimethyl 2-(4-chlorobenzylidene)malonate, dimethyl 2-(4-fluorobenzylidene)malonate, dimethyl 2-(4-ethylbenzylidene)malonate, dimethyl 2-(4-isopropylbenzylidene)malonate, dimethyl 2-(4-tert-butylbenzylidene)malonate, dimethyl 2-(4-isobutylbenzylidene)malonate, dimethyl 2-(2,4,6-trimethylbenzylidene)malonate, or diisopropyl 2-(4-isopropylbenzylidene)malonate.

### (Ultraviolet Absorber)

The additive of the present invention is useful as an ultraviolet absorber for efficiently absorbing ultraviolet light of, particularly, a UV-B region (280 to 320 nm) which is believed to cause deterioration of resins. The additive of the present invention exhibits a maximum absorption wavelength of 280 to 310 nm, preferably 280 to 300 nm, more preferably 280 to 290 nm.

### (Method of manufacturing additive)

The method of manufacturing the additive according to the present invention is not particularly limited, but an example of which includes a method of making condensation reaction between a benzaldehyde derivative and a compound containing a carbon atom having a highly acidic hydrogen atom that is sandwiched between two carbonyl carbons of, for example, malonic acid diester or barbituric acid.

### (Use of Additive)

The additive of the present invention may be added to an organic or inorganic material. Examples of the organic material include, but are not particularly limited to, organic compounds of, for example, a resin, a material of plant or animal origin, and/or a material of crude oil origin, while examples of the inorganic material include, but are not particularly limited to, for example, inorganic compounds of, for example, sol-gel-based siliceous materials, glass, liquid glass, low-melting-point glass, quartz, silicon resin, alkoxysilane, silane coupling agents, metals, metal oxides, and/or minerals. Of these, they may be suitably used for absorbing or suppressing transmission of the UV-B region. For example, they are useful for improving light resistance or preventing deterioration of resins and suppressing UV-B region transmission through a transparent member of, for example, glass or resin.

The additive of the present invention has the advantage that it volatilizes and decomposes little under heat when added to resins for use. The additive of the present invention can be used alone or in combination of two or more.

A conventional method of adding an additive to resin may be applied thereto. Examples of such method that can be used include, but are not particularly limited to, a method of mixing the additive into a resin solution, a method of melting a resin and mixing the additive into it, and a method of mixing the additive into raw material monomer of resin and then resinifying the same.

That is, the additive of the present invention may be added to a resin to make a resin composition.

Examples of such resin to which the additive of the present invention may be added include, but are not particularly limited to, for example, thermoplastic and thermosetting resins each including a polymer having one type of repeating unit or a copolymer having multiple repeating units, and a wide variety of conventionally known resins may be used therefor.

Of these resins, preferred is a thermoplastic resin. The types of thermoplastic resin are not particularly limited. Examples of the polymer include a (meth)acrylic-based resin, an olefin-based resin, a styrene-based resin, an ester-based resin, an ether-based resin, a vinyl chloride-based resin, a fluorocarbon-based resin, a vinyl-based resin, a polycarbonate-based resin, a polyamide-based resin, a polyimide-based resin, a polyamideimide-based resin, a polymaleimide-based resin, a polyvinylpyrrolidone-based resin, a polyurethane-based resin, a polysulfone-based resin, a polyphenylene sulfide-based resin and a cycloolefin-based resin. Examples of the copolymer include a butadiene-styrene-based copolymer, an acrylonitrile-styrene-based copolymer, an acrylonitrile-butadiene-styrene-based copolymer, a styreneisoprene-based copolymer, a styrene-acrylic acid-based copolymer and a vinyl chloride-vinylidene chloride-acrylonitrile-based copolymer.

Of these, preferred are a (meth)acrylic-based resin (such as acrylic resin), a styrene-based resin (such as polystyrene), an ester-based resin (such as polyethylene terephthalate resin), an ether-based resin (such as polyacetal), a polycarbonate-based resin (such as polycarbonate), a polyamide-based resin (such as polyamide), a polyphenylene sulfide-based resin, a cycloolefin-based resin (such as cycloolefin polymer), and an acrylonitrile-butadiene-styrene-based copolymer (such as acrylonitrile-butadiene-styrene copolymer resin).

When an additive is mixed or kneaded with an organic or inorganic substance such as resin under heating, or when a resin member containing an additive is processed or molded by heating, an additive having low heat resistance undergoes thermal decomposition or thermal denaturation, and the ultraviolet absorbing effect will not be fully exerted, contaminating the equipment, and if the resin is transparent, it will lose its transparency. In addition, the transmittance in the visible light region decreases and coloration occurs due to, for example, thermal decomposition or thermal denaturation. Accordingly, desired is an additive that suppresses coloration during heating, has a high decomposition onset temperature during heating, and has excellent heat resistance.

The additive of the present invention is excellent in heat resistance (suppresses coloration during heating and has a high decomposition onset temperature), suppresses coloration and functional deterioration of the additive under high temperature, thus capable of being favorably used for a resin having a thermoforming or thermosetting temperature of 100°C or higher, particularly 200°C or higher, or for manufacturing or using the resin at the above-mentioned temperature. The additive of the present invention may therefore be favorably used for a thermoplastic resin polymer or copolymer, particularly a thermoplastic resin polymer or copolymer having the above-mentioned thermoforming temperature.

For example, among the thermoplastic resin polymers, it can be preferably used for a styrene-based resin (polystyrene: PS, molding temperature of 100°C), a (meth)acrylic-based resin (polymethyl methacrylate, molding temperature of 160°C), a cycloolefin-based resin (molding temperature of 100°C), a polycarbonate-based resin (polycarbonate: PC, molding temperature of 200°C or higher), and an ester-based resin (polyethylene terephthalate: PET, molding temperature of 200°C or higher).

In addition, among the thermoplastic resin copolymers, it can be preferably used for an acrylonitrile-butadiene-styrene-based copolymer (acrylonitrile-butadiene-styrene copolymer: ABS, molding temperature of 200°C or higher).

A further component may be added to the additive of the present invention so long as the component does not impair the advantageous effect of the present invention. Examples of the further component include, but are not particularly limited to, a solvent, a light stabilizer, an antioxidant, and a pH adjuster.

### Examples

The present invention is described in greater detail hereunder with reference to working examples. However, the invention shall not be limited to the following working examples.

### 1. Synthesis of Additive Compound

Compounds 1 to 8 were synthesized using the methods explained below.

### <Compound 1> (MYUA-1M)

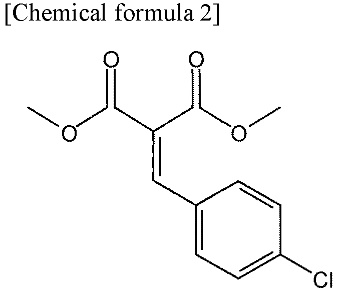

4-chlorobenzaldehyde (7.03 g), dimethyl malonate (6.61 g), piperidine (421 mg), and acetic acid (605 mg) were stirred under reflux in 20 mL of toluene while distilling away the water produced by reaction until the generation of water could no longer be confirmed. After the reaction was completed, the organic layer was washed with water and purified by column chromatography to obtain a compound 1.
IR (ATR): 1726 cm⁻¹(C=O stretch)
¹HNMR (CDCl₃, 400 MHz): 7.72(s,1H), 7.36(s,4H), 3.85(s,3H), 3.85(s,3H)
¹³CNMR (CDCl₃, 400 MHz): 166.9, 164.3, 141.6, 141.4, 131.3, 130.8, 130.4, 129.4, 129.0, 126.1, 53.3, 52.4

### <Compound 2>

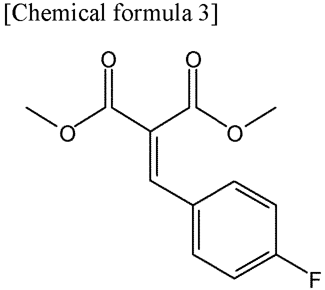

4-fluorobenzaldehyde (6.21 g), dimethyl malonate (6.61 g), piperidine (421 mg), and acetic acid (605 mg) were stirred under reflux in 20 mL of toluene while distilling away the water produced by reaction until the generation of water could no longer be confirmed. After the reaction was completed, the organic layer was washed with water and purified by column chromatography to obtain a compound 2.
IR (ATR): 1729 cm⁻¹(C=O stretch)
¹HNMR (CDCl₃, 400 MHz): 7.73(s,1H), 7.43(q,2H), 7.08(t,2H), 3.85(s,3H), 3.85(s,3H)
¹³CNMR (CDCl₃, 400 MHz): 167.0, 164.4, 141.6, 141.4, 131.5, 129.0, 129.0, 125.3, 116.5, 115.9, 53.1, 52.4

### <Compound 3>

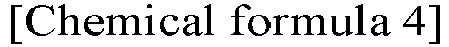

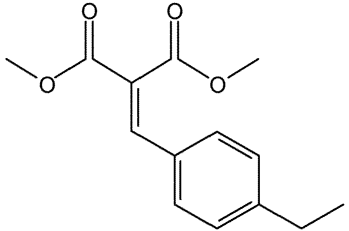

4-ethylbenzaldehyde (6.71 g), dimethyl malonate (6.61 g), piperidine (421 mg), and acetic acid (605 mg) were stirred under reflux in 20 mL of toluene while distilling away the water produced by reaction until the generation of water could no longer be confirmed. After the reaction was completed, the organic layer was washed with water and purified by column chromatography to obtain a compound 3.
IR (ATR): 1735 cm⁻¹(C=O stretch)
¹HNMR (CDCl₃, 400 MHz): 7.75(s,1H), 7.35(d,2H), 7.21(d,2H), 3.86(s,3H), 3.84(s,3H), 2.66(q, 2H), 1.24(t,3H)
¹³CNMR (CDCl₃, 400 MHz): 167.4, 164.7, 147.6, 143.0, 130.1, 129.8, 129.5, 128.7, 128.3, 124.3, 53.0, 52.3, 28.8, 15.2

### <Compound 4>

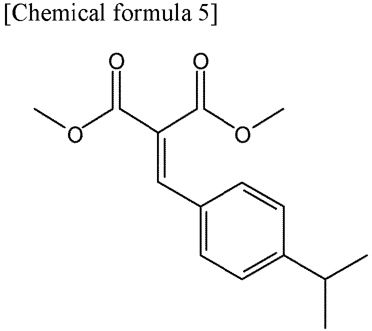

Cuminaldehyde (4.97 g), dimethyl malonate (4.43 g), piperidine (282 mg), and acetic acid (406 mg) were stirred under reflux in 20 mL of toluene while distilling away the water produced by reaction until the generation of water could no longer be confirmed. After the reaction was completed, the organic layer was washed with water and purified by column chromatography to obtain a compound 4.
IR (ATR): 1729 cm⁻¹(C=O stretch)
¹HNMR (CDCl₃, 400 MHz): 7.74(s,1H), 7.36(d,2H), 7.24(d,2H), 3.87(s,3H), 3.84(s,3H), 2.92(m,1H), 1.25(s,3H), 1.24(s,3H)
¹³CNMR (CDCl₃, 400 MHz): 167.4, 164.7, 152.2, 142.9, 130.2, 129.7, 129.3, 127.3, 126.9, 124.3, 53.0, 52.3, 34.1, 24.0, 23.4

### <Compound 5>

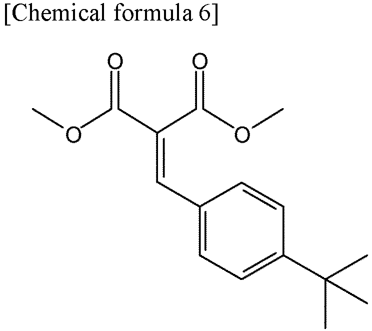

4-tert-butylbenzaldehyde (8.12g), dimethyl malonate (6.61 g), piperidine (421 mg), and acetic acid (605 mg) were stirred under reflux in 20 mL of toluene while distilling away the water produced by reaction until the generation of water could no longer be confirmed. After the reaction was completed, the organic layer was washed with water and purified by column chromatography to obtain a compound 5.
IR (ATR): 1726 cm⁻¹(C=O stretch)
¹HNMR (CDCl₃, 400 MHz): 7.74(s,1H), 7.36-7.42(m,4H), 3.87(s,3H), 3.84(s,3H), 1.32(s,9H)
¹³CNMR (CDCl₃, 400 MHz): 167.5, 164.7, 154.5, 142.8, 129.8, 129.7, 129.4, 126.1, 125.8, 124.3, 53.7, 53.0, 52.3, 35.0, 31.3, 30.8

### <Compound 6>

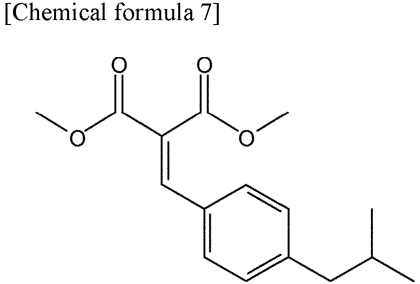

4-isobutylbenzaldehyde (8.11 g), dimethyl malonate (6.61 g), piperidine (421 mg), and acetic acid (605 mg) were stirred under reflux in 20 mL of toluene while distilling away the water produced by reaction until the generation of water could no longer be confirmed. After the reaction was completed, the organic layer was washed with water and purified by column chromatography to obtain a compound 6.
IR (ATR): 1738 cm⁻¹(C=O stretch)
¹HNMR (CDCl₃, 400 MHz): 7.75(s,1H), 7.34(d,2H), 7.16(d,2H), 3.86(s,3H), 3.84(s,3H), 2.49(d,2H), 1.87(m,1H), 0.91(s,3H), 0.89(s,3H)
¹³CNMR (CDCl₃, 400 MHz): 167.4, 164.7, 145.2, 143.0, 130.1, 130.0, 129.6, 129.5, 129.3, 124.2, 53.0, 52.3, 45.3, 30.2, 22.6, 22.1

### <Compound 7>

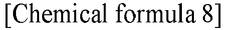

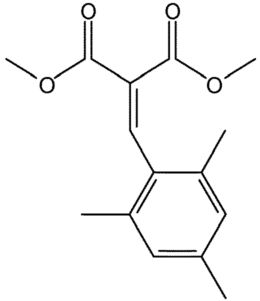

Mesitaldehyde (7.41 g), dimethyl malonate (6.61 g), piperidine (421 mg), and acetic acid (605 mg) were stirred under reflux in 20 mL of toluene while distilling away the water produced by reaction until the generation of water could no longer be confirmed. After the reaction was completed, the organic layer was washed with water and purified by column chromatography to obtain a compound 7.
IR (ATR): 1738 cm⁻¹(C=O stretch)
¹HNMR (CDCl₃, 400 MHz): 7.90(s,1H), 6.83(s,2H), 3.87(s,3H), 3.57(s,3H), 2.26(s,3H), 2.16(s,6H),
¹³CNMR (CDCl₃, 400 MHz): 165.6, 164.2, 146.4, 146.2, 137.9, 135.0, 130.2, 128.4, 127.7, 124.2, 52.9, 52.3, 20.2, 19.9

### <Compound 8>

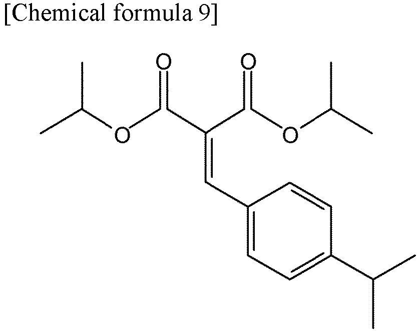

Cuminaldehyde (14.8 g), diisopropyl malonate (18.8 g), piperidine (850 mg), and acetic acid (1.21 g) were stirred under reflux in 40 mL of toluene while distilling away the water produced by reaction until the generation of water could no longer be confirmed. After the reaction was completed, the organic layer was washed with water and purified by column chromatography to obtain a compound 8.
IR (ATR): 1739 cm⁻¹(C=O stretch)
¹HNMR (CDCl₃, 400 MHz): 7.65(s,1H), 7.41(d,2H), 7.22(d,2H), 5.27(m,1H), 5.15(m,1H), 2.91(m,1H), 1.31(d,12H), 1.24(d,6H)
¹³CNMR (CDCl₃, 400 MHz): 166.5, 163.9, 151.8, 141.4, 130.5, 129.9, 129.6, 127.1, 126.7, 125.9, 69.2, 69.1, 34.2, 24.0, 23.4, 22.1, 21.8, 21.5, 21.3

### <Compound 9>

A reagent manufactured by Tokyo Chemical Industry Co., Ltd. was used as 2-(4-methoxybenzylidene)dimethyl malonate.

### 2. Measurement of Ultraviolet Absorption Wavelength

For each of the compounds 1 to 9, a 100 µM chloroform solution was prepared, and the absorption spectrum was measured using an UV-Vis-NIR spectrophotometer (UH4150V, manufactured by Hitachi High-Tech Science Corporation) to confirm the maximum absorption wavelength (Table 1).

The compounds 1 to 8 of the working examples had maximum absorption wavelengths at 280 to 310 nm in the UV-B region (280 to 320 nm) as shown in the maximum absorption wavelengths in Table 1, which demonstrates that the compounds 1 to 8 are superior to the compound 9 of the comparative example in that they can efficiently absorb the UV-B region.

It was also indicated from this fact that the additive of the present invention is useful as an ultraviolet absorber, and particularly the compounds 1 to 8 of the working examples in which each of R³ to R⁷ has a hydrogen atom, hydrocarbon group, or a halogen are useful because they exhibit maximum absorption wavelengths at 280 to 300 nm. It was further indicated that the compounds 1 and 2 of the working examples in which R³ to R⁷ have a hydrogen atom and a halogen are particularly useful because they exhibit maximum absorption wavelengths at 280 to 290 nm.

### 3. State at 25°C

The states of the compounds 1 to 9 at 25°C were visually observed and recorded as either liquid or solid in Table 1. It was observed from the working examples 1 and 2 that the compounds in which R⁵ was chlorine or fluorine became solid. This fact indicates that the compounds in which R⁵ is a halogen become solid at 25°C.

It was indicated from the working example 5 that compounds having a quaternary carbon, such as a 2-methylpropan-2-yl group, which is a relatively bulky functional group, became solid at 25°C, and it was also indicated that from the working example 7 that compounds having three or more hydrocarbon groups in R³ to R⁷ became solid at 25°C.

### 4. Heat Resistance Test (1)

Compounds 1 to 8 (1 mmol) were each placed in a 30 mL sample bottle and heated in air for 10 minutes in a muffle furnace set to 300°C. After cooling, 10 mL of chloroform was added and the presence or absence of coloring was visually observed to evaluate them as follows, and the results are shown in Table 1.
No coloring was observed after heating: ⊚
Compound was slightly colored after heating: ∘
Compound was colored in brown after heating: △
Compound was colored in black after heating: ×

The test results showed that in comparison with compounds 1 to 8, compounds in which R³ to R⁷ had a halogen (compounds 1 and 2) suppress coloring after heating compared to those in which R³ to R⁷ had a hydrocarbon group (compounds 3 to 8), and it was particularly confirmed that compounds in which the halogen was a chlorine atom exhibited an excellent heat resistance.

These results suggest that different decomposition mechanisms work between the compounds in which R³ to R⁷ consist of hydrogen atom(s) and halogen(s) and the compounds in which R³ to R⁷ consist of hydrogen atom(s) and hydrocarbon group(s), and that colored decomposition products are less likely to be produced when R³ to R⁷ consist of hydrogen atom(s) and halogen(s).

### 5. Heat Resistance Test (2)

A simultaneous thermogravimetric analyzer (STA7200 manufactured by Hitachi High-Tech Science Corporation) was applied to the compounds 1 to 8 of the present invention to measure the weight differences at a temperature rise rate of 10°C/min under a nitrogen atmosphere in a measurement range of 25°C to 550°C, and the decomposition onset temperature was calculated from the endothermic peak of the differential thermal analysis (DTA). The results are shown in Table 1.

It was confirmed that the whole compounds 1 to 8 had decomposition onset temperatures of 190°C or higher, and therefore had excellent heat resistances. In particular, the compounds 1, 3 to 6, and 8 had decomposition onset temperatures of 210°C or higher, showing superior results in heat resistance.

### 6. Change in Transmittance in UV-B region (300 nm) upon Heating

Compounds 1 and 9 (0.1 mmol) were respectively placed in 30 mL sample bottles and heated for 10 minutes in a muffle furnace set at 300°C. After cooling, 10 mL of chloroform was added and the transmittance at 300 nm was measured. The change in transmittance at each wavelength was calculated by comparing it with the transmittance of the compounds 1 and 9 prior to heating.

Both compounds 1 and 9 exhibited the transmittance at 300 nm of 0% before heating, whereas, after heating, compound 1 remained unchanged at 0%, while the transmittance of the compound 9 became 47%.

This demonstrates that the compound 9 had a decreased UV absorption capability after being heated, whereas the compound 1 remained intact after heating in respect of UV absorption ability.

These results indicate that the additive in the present invention demonstrates outstanding heat resistance, as it effectively prevents the degradation of UV absorption capability caused by heat.

**[Table 1]**

| | Compound | R¹ | R² | R³ | R⁴ | R⁵ | R⁶ | R⁷ | Maximum Absorption Wavelength (nm) | State at 25°C | Heat resistance test (1) (Coloration) | Heat resistance test (2) (Decomposition Onset Temperature : °C ) |
|---|---|---|---|---|---|---|---|---|---|---|---|---|
| Working Example 1 | 1 | -O-Me | -O-Me | H | H | -Cl | H | H | 289 | Solid | ⊚ | 214 |
| Working Example 2 | 2 | -O-Me | -O-Me | H | H | -F | H | H | 283 | Solid | ○ | 193 |
| Working Example 3 | 3 | -O-Me | -O-Me | H | H | -Et | H | H | 295 | Liquid | △ | 219 |
| Working Example 4 | 4 | -O-Me | -O-Me | H | H | -iPr | H | H | 292 | Liquid | △ | 220 |
| Working Example 5 | 5 | -O-Me | -O-Me | H | H | -tBu | H | H | 296 | Solid | △ | 231 |
| Working Example 6 | 6 | -O-Me | -O-Me | H | H | -CH₂-iPro | H | H | 296 | Liquid | △ | 221 |
| Working Example 7 | 7 | -O-Me | -O-Me | Me | H | -Me | H | Me | 291 | Solid | △ | 197 |
| Working Example 8 | 8 | -O-iPro | -O-iPro | H | H | -iPro | H | H | 292 | Liquid | △ | 228 |
| Comparative Example 1 | 9 | -O-Me | -O-Me | H | H | -O-Me | H | H | 315 | Solid | - | - |

## Claims

1. An additive represented by the following general formula (1): 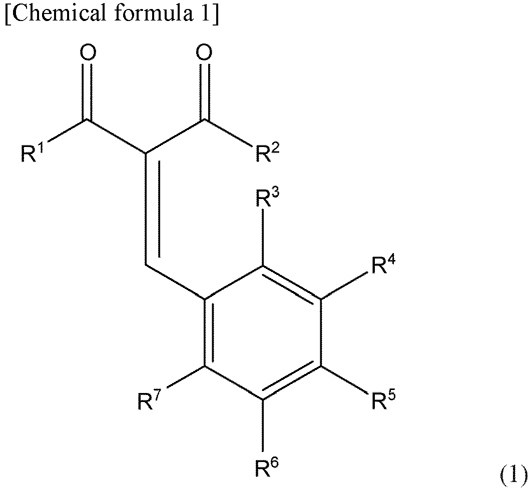 wherein, in the formula, R¹ and R² each independently represent an alkoxy group having 1 to 10 carbon atoms, or are bonded to a divalent group to form a ring, and R³ to R⁷ independently represent at least one selected from a hydrogen atom, a hydrocarbon group having 1 to 10 carbon atoms, a sulfur-containing group, a nitrogen-containing group, and a halogen, or any two among R³ to R⁷ are bonded to a divalent group to form a ring.

2. The additive according to claim 1, wherein the additive is an ultraviolet absorber.

3. The additive according to claim 2, wherein the additive exhibits a maximum absorption wavelength of 300 nm or less in a 100 µM solution.
